# EUROPEAN PATENT APPLICATION

(11) **EP 3 378 373 A1**
(43) Date of publication of application: **26.09.2018**
(21) Application number: 15908819.4
(22) Date of filing: 20.11.2015
(51) Int. Cl.: A61B 1/00

(54) **VARIABLE HARDNESS ACTUATOR**

(71) Applicant: Olympus Corporation, Hachioji-shi, Tokyo 192-8507 (JP)
(72) Inventor: MORISHIMA, Tetsuya, Tokyo 192-8507 (JP)
(74) Representative: Winter, Brandl, Fürniss, Hübner, Röss, Kaiser, Polte - Partnerschaft mbB
(86) International application number: PCT/JP2015/082754
(87) International publication number: WO 2017/085880

(57) **Abstract**

A variable-stiffness actuator (10) that is to be installed in a flexible member and configured to provide different stiffnesses to the flexible member includes a shape-memory member (20) configured to transit in phase between a first phase and a second phase. The shape-memory member (20) takes a flexible state in which the shape-memory member is easily deformable by an external force when the shape-memory member is in the first phase, so as to provide lower stiffness to the flexible member. The shape-memory member (20) also takes a rigid state in which the shape-memory member tends to take a memorized shape memorized beforehand against an external force when the shape-memory member is in the second phase, so as to provide higher stiffness to the flexible member. The variable-stiffness actuator (10) further includes a conductive inducing member (30) that induces phase transition of the shape-memory member between the first phase and the second phase, and a conductive member (70) electrically connected to the inducing member (30). The conductive member (70) is arranged around an outside of the inducing member and physically protects the inducing member.

## Description

### FIELD

The present invention relates to a variable-stiffness actuator for varying the stiffness of a flexible member.

### BACKGROUND

Japanese Patent No. 3122673 discloses an endoscope capable of varying the stiffness of a flexible section of an insertion section. In this endoscope, both ends of a flexible member (such as a coil pipe) are fixed at predetermined positions in the endoscope, and a flexibility adjustment member (such as a flexibility adjustment wire inserted through a coil pipe) is fixed to the flexible member through a separator. The flexible member and the flexibility adjustment member extend to a handling section along the flexible section and extend almost all over the flexible section. The flexible member is compressed and stiffened by pulling the flexibility adjustment member, thereby varying the stiffness of the flexible section.

Since the flexible member and the flexibility adjustment member extend almost all over the flexible section, significantly more power is required to drive such a mechanism. In order to motorize the mechanism, a large power source is required, which results in an extensive configuration.

Japanese Patent No. 3142928 discloses a variable-stiffness apparatus for flexible tubes using a shape-memory alloy. The variable-stiffness apparatus includes a coil to be provided in a flexible tube, an electrical insulating tube provided inside the coil, a shape-memory alloyed wire arranged in the electrical insulating tube so as to extend in its axial direction, and an energization heating means for energizing the shape-memory alloyed wire.

The shape-memory alloyed wire has properties of elongating at a low temperature and contracting at a high temperature. The shape-memory alloyed wire extends out through fixed portions at both ends of the coil, and caulking members are fixed to the both ends. The shape-memory alloyed wire is arranged so that it loosens at a low temperature and it tightens up at a high temperature with the caulking members engaged with the fixed portions.

The shape-memory alloyed wire contracts to stiffen the coil at a high temperature at which it is energized by the energization heating means. On the other hand, the shape-memory alloyed wire elongates to soften the coil at a low temperature at which it is not energized.

This variable-stiffness apparatus has a simple configuration, and thus may be compact; however, when the shape-memory alloyed wire contracts, the both ends of the shape-memory alloyed wire are restricted, and a load is imposed on the shape-memory alloyed wire. Accordingly, its durability is a problem.

### SUMMARY

An object of the present invention is provide a variable-stiffness actuator includes that is to be installed in a flexible member, configured to provide different stiffnesses to the flexible member, simple in structure, and has durability.

For this object, a variable-stiffness actuator includes a shape-memory member configured to transit in phase between a first phase and a second phase. The shape-memory member takes a flexible state in which the shape-memory member is easily deformable by an external force when the shape-memory member is in the first phase, so as to provide lower stiffness to the flexible member. The shape-memory member also takes a rigid state in which the shape-memory member tends to take a memorized shape memorized beforehand against an external force when the shape-memory member is in the second phase, so as to provide higher stiffness to the flexible member. The variable-stiffness actuator further includes a conductive inducing member that induces phase transition of the shape-memory member between the first phase and the second phase, and a conductive member electrically connected to the inducing member. The conductive member is arranged around an outside of the inducing member and physically protects the inducing member.

### BRIEF DESCRIPTION OF THE DRAWINGS

FIG. 1 shows a variable-stiffness actuator according to a first embodiment.
FIG. 2 shows a variable-stiffness actuator according to a second embodiment.
FIG. 3 shows a variable-stiffness actuator according to a third embodiment.
FIG. 4 shows a variable-stiffness actuator according to a fourth embodiment.
FIG. 5 shows a variable-stiffness actuator according to a fifth embodiment.

### DESCRIPTION OF EMBODIMENTS

### [First Embodiment]

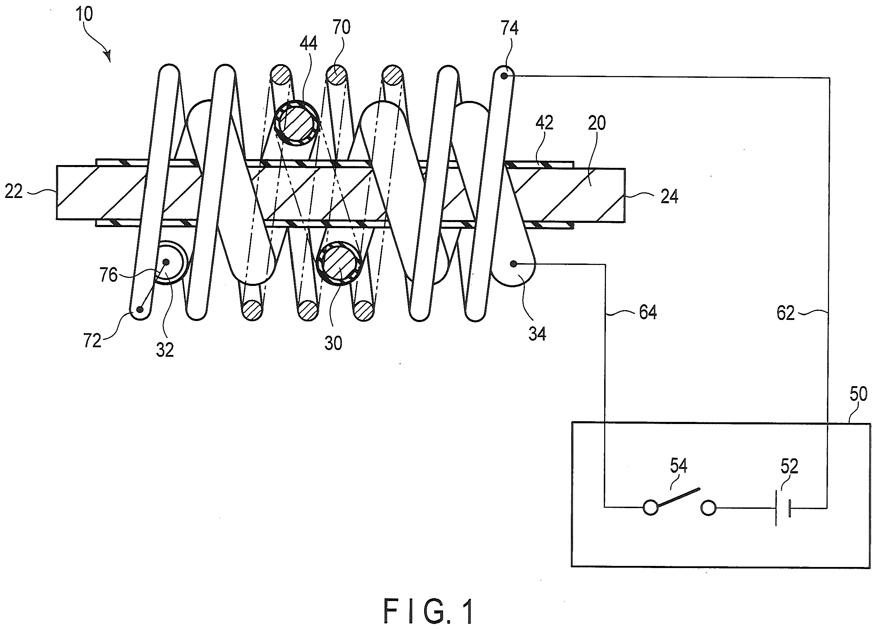

FIG. 1 shows a variable-stiffness actuator according to a first embodiment. As shown in FIG. 1, a variable-stiffness actuator 10 has a function of providing different stiffnesses for a flexible member by taking different stiffness states, and includes a shape-memory member 20 that can transit in phase between a first phase and a second phase, an inducing member 30 that induces phase transition of the shape-memory member 20 between the first phase and the second phase, and a conductive member 70 electrically connected to the inducing member 30. The shape-memory member 20 is arranged in the flexible member with at least one free end.

The shape-memory member 20 takes a flexible state in which it is easily deformable by an external force, i.e., exhibits a low elastic modulus, when it is in the first phase, so as to provide lower stiffness to the flexible member. The shape-memory member 20 takes a rigid state in which it tends to take a memorized shape memorized beforehand against an external force, i.e., exhibits a high elastic modulus, when it is in the second phase, so as to provide higher stiffness to the flexible member. The memorized shape may be, but is not limited to, a linear shape.

Herein, the external force means a force that can deform the shape-memory member 20, and gravity is considered to be part of the external force.

The inducing member 30 has the capability of generating heat. The shape-memory member 20 has the property of transiting in phase from the first phase to the second phase in response to heating of the inducing member 30.

The shape-memory member 20 may be made of, for example, a shape-memory alloy. The shape-memory alloy may be an alloy including, but not limited to, NiTi, for example. The shape-memory member 20 is not limited to the above, and may also be made of another material, such as a shape-memory polymer, shape-memory gel, or shape-memory ceramics.

The shape-memory alloy forming the shape-memory member 20 may be, for example, a shape-memory alloy that transits in phase between a martensitic phase and an austenitic phase. In the martensitic phase, the shape-memory alloy is plastically deformed relatively easily by an external force. In other words, the shape-memory alloy exhibits a low elastic modulus in the martensitic phase. In the austenitic phase, the shape-memory alloy is not easily deformed by an external force. Even when the shape-memory alloy is deformed by a greater external force, it exhibits superelasticiy and returns to its memorized shape when the greater external force is lost. In other words, the shape-memory alloy exhibits a high elastic modulus in the austenitic phase.

The inducing member 30 is formed by a conductive material, and has the property of generating heat in response to supply of a current. The inducing member 30 may be formed by, for example, a heating wire, i.e., a conductive member with large electrical resistance.

The shape-memory member 20 has a slim exterior shape. The inducing member 30 is formed by a member shaped like a wire, and is arranged around the outside of the shape-memory member 20. The inducing member 30 spirally extends around the shape-memory member 20 along the longitudinal axis of the shape-memory member 20 with an appropriate clearance from the shape-memory member 20. This configuration enables efficient conduction of heat generated by the inducing member 30 to the shape-memory member 20.

In contrast to the inducing member 30, the conductive member 70 may be formed by a conductive material with small electrical resistance. The conductive member 70 is formed by a member shaped like a wire, and is arranged around the outside of the inducing member 30. The conductive member 70 spirally extends around the inducing member 30 along the longitudinal axis of the shape-memory member 20 with an appropriate clearance from the inducing member 30. This layout enables the conductive member 70 to physically satisfactory protect the inducing member 30.

The shape-memory member 20 may be formed by a conductive material. For example, an insulating film 42 is provided around the shape-memory member 20. The insulating film 42 serves to prevent a short circuit between the shape-memory member 20 and the inducing member 30. The insulating film 42 is provided to cover at least a part facing the inducing member 30. FIG. 1 shows a configuration in which the outer peripheral surface of the shape-memory member 20 is partly covered; however, the configuration is not limited to this, and the outer peripheral surface of the shape-memory member 20 may be entirely covered, or the shape-memory member 20 may be entirely covered.

An insulating film 44 is provided around the inducing member 30. The insulating film 44 serves to prevent a short circuit between the shape-memory member 20 and the inducing member 30 and a short circuit between adjacent portions of the inducing member 30.

The shape-memory member 20 has a first end 22 and a second end 24, and the inducing member 30 has a first end 32 located on a side of the first end 22 of the shape-memory member 20 and a second end 34 located on a side of the second end 24 of the shape-memory member 20. The conductive member 70 has a first end 72 located on a side of the first end 22 of the shape-memory member 20 and a second end 74 located on a side of the second end 24 of the shape-memory member 20.

The first end 32 of the inducing member 30 is electrically connected to the first end 72 of the conductive member 70 through a connector 76. The connector 76 may be formed by, for example, a wire, but is not limited thereto. As long as the connector 76 is a structure enabling electrical connection, it may be formed by calking, welding, brazing, soldering, conductive adhesion, or the like.

The second end 34 of the inducing member 30 is electrically connected to a controller 50 through a wire 64. The second end 74 of the conductive member 70 is electrically connected to the controller 50 through a wire 62.

The controller 50 includes a power source 52 and a switch 54. One end of the switch 54 is connected to wire 64, and the other end of the switch 54 is connected to the power source 52. Wire 62 is also connected to the power source 52. The controller 50 supplies a current to the inducing member 30 in response to ON, i.e., a closing operation, of the switch 54, and stops supplying a current to the inducing member 30 in response to OFF, i.e., an opening operation of the switch 54. The inducing member 30 generates heat in response to supply of a current.

The above-described variable-stiffness actuator 10 is installed in the flexible member without restricting both ends of the shape-memory member 20. For example, the variable-stiffness actuator 10 is arranged in a limited space of the flexible member with a small clearance so that an end or both ends of the shape-memory member 20 are a free end or free ends.

Herein, the limited space means space of a right size capable of containing the variable-stiffness actuator 10 therein. Thus, even when one of the variable-stiffness actuator 10 and the flexible member is slightly deformed, it can contact the other and give an external force.

For example, the flexible member may be a tube having an inner diameter slightly larger than the outer diameter of the variable-stiffness actuator 10, and the variable-stiffness actuator 10 may be placed inside the tube. The configuration of the flexible member is not limited to this, and the flexible member only has to have a slightly larger space than the variable-stiffness actuator 10.

When the shape-memory member 20 is in the first phase, the variable-stiffness actuator 10 provides lower stiffness to the flexible member and is easily deformed by an external force exerted on the flexible member, i.e., a force capable of deforming the shape-memory member 20.

When the shape-memory member 20 is in the second phase, the variable-stiffness actuator 10 provides higher stiffness to the flexible member and tends to return to its memorized shape against an external force exerted on the flexible member, i.e., a force capable of deforming the shape-memory member 20.

For example, as the phase of the shape-memory member 20 is switched between the first and second phases by the controller 50, the stiffness of the flexible member is switched.

In addition to switching the stiffness, in a situation where an external force is exerted on the flexible member, the variable-stiffness actuator 10 also functions as a bidirectional actuator that switches the shape of the flexible member. In another situation where no external force is exerted on the flexible member, but the flexible member is deformed in the first phase before the phase of the shape-memory member 20 is switched to the second phase, the variable-stiffness actuator 10 also serves as a unidirectional actuator that returns the shape of the flexible member to the original.

### [Second Embodiment]

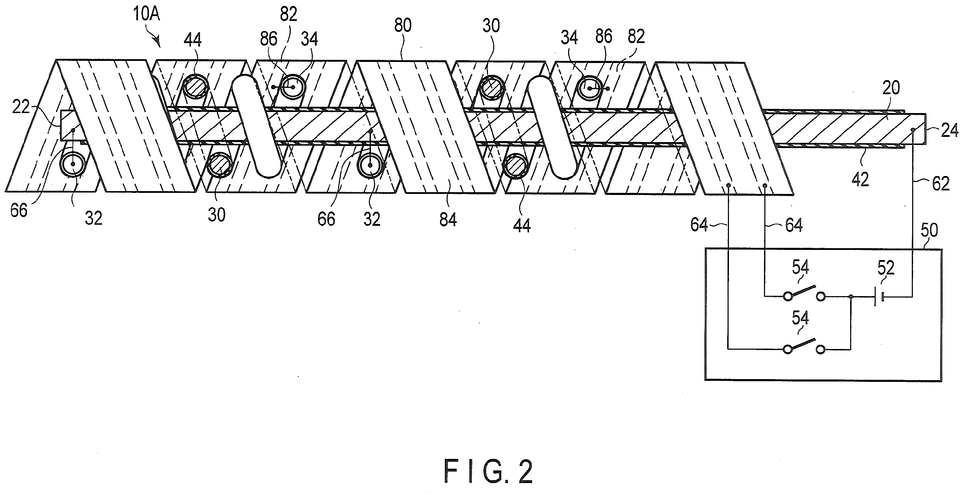

FIG. 2 shows a variable-stiffness actuator according to a second embodiment. In FIG. 2, the same members as those shown in FIG. 1 are assigned the same reference numerals as those shown in FIG. 1, and detailed descriptions thereof are omitted. The following descriptions will be provided while placing importance on the parts different from those in FIG. 1. Namely, the parts not described below are the same as those in the first embodiment.

As shown in FIG. 2, the variable-stiffness actuator 10A includes a thin shape-memory member 20 and a plurality of inducing members 30. The variable-stiffness actuator 10A shown in FIG. 2 includes two inducing members 30, but is not limited to this, and may include more inducing members 30. The inducing members 30 are arranged at intervals along the longitudinal axis of the shape-memory member 20. Each inducing member 30 spirally extends around the shape-memory member 20 along the longitudinal axis of the shape-memory member 20 with an appropriate clearance from the shape-memory member 20. This configuration enables efficient conduction of heat generated by the inducing members 30 to the shape-memory member 20.

The inducing members 30 may be the same structure. However, the inducing members 30 are not limited to this, and may include different structures. The different structures may have different lengths, diameters, or pitches, and may be made of different materials. Namely, all or some of the inducing members 30 may have the same characteristics or different characteristics.

The variable-stiffness actuator 10A further includes an outside wire 80 arranged around the outsides of the inducing members 30. The outside wire 80 is shaped like a belt, and includes a plurality of conductive members 82 and a thin insulating layer 84 supporting the conductive members 82. The number of the conductive members 82 may be for example, the same as the number of inducing members 30, but is not limited to this, and may be more than the number of inducing members 30. The conductive members 82 extend in the longitudinal direction of the insulating layer 84. The conductive members 82 are, for example, embedded in the insulating layers 84. The outside wire 80 spirally extends around the outsides of the inducing members 30. This layout enables the outside wire 80 to physically satisfactory protect the inducing members 30.

Each inducing member 30 has a first end 32 located on a side of the first end 22 of the shape-memory member 20 and a second end 34 located on a side of the second end 24 of the shape-memory member 20.

The shape-memory member 20 also has electrical conductivity. The first ends 32 of the inducing members 30 are electrically connected to the shape-memory member 20 through connectors 66. Each connector 66 may be formed by, for example, a wire, but is not limited thereto. As long as the connector 66 is a structure enabling electrical connection, it may be formed by calking, welding, brazing, soldering, conductive adhesion, or the like. The shape-memory member 20 is electrically connected on a side of the second end 24 to the controller 50 through a wire 62.

The second ends 34 of the inducing members 30 are electrically connected to the conductive members 82 of the outside wire 80 through connectors 86, respectively. The conductive members 82 are electrically connected to the controller 50 through wires 64, respectively.

The controller 50 includes a power source 52 and a plurality of switches 54. One ends of the switches 54 are connected to wires 64, and the other ends of the switches 54 are connected to the power source 52 in common. Wire 62 is also connected to the power source 52. The controller 50 independently supplies a current to the inducing members 30 in response to ON, i.e., closing operations, of the corresponding switches 54, and stops supplying a current to the inducing members 30 in response to OFF, i.e., opening operations of the corresponding switches 54. The inducing members 30 generate heat in response to supply of a current.

The variable-stiffness actuator 10A of the present embodiment is installed in the same manner as the variable-stiffness actuator 10 of the first embodiment. In the variable-stiffness actuator 10A of the present embodiment, the inducing members 30 are independently operated in response to ON/OFF of the corresponding switches 54. The actuator operation by each inducing member 30 is the same as that in the first embodiment.

### [Third Embodiment]

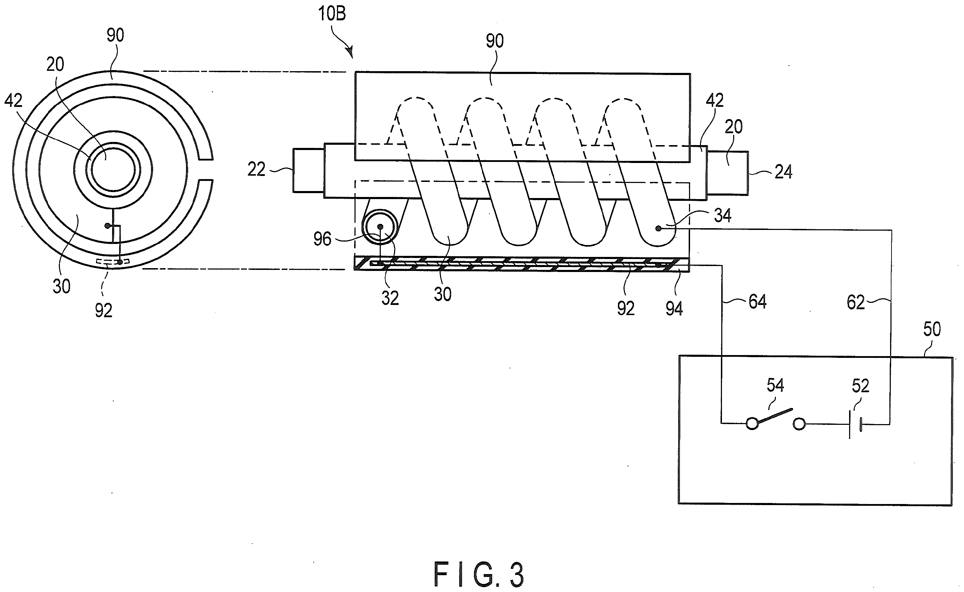

FIG. 3 shows a variable-stiffness actuator according to a third embodiment. In FIG. 3, the same members as those shown in FIG. 1 are assigned the same reference numerals as those shown in FIG. 1, and detailed descriptions thereof are omitted. The following descriptions will be provided while placing importance on the parts different from those in FIG. 1. Namely, the parts not described below are the same as those in the first embodiment.

As shown in FIG. 3, the variable-stiffness actuator 10B includes a thin shape-memory member 20, an inducing member 30 spirally extending around the outside of the shape-memory member 20, and an outside wire 90 arranged around the outside of the inducing member 30.

The outside wire 90 is shaped like a belt or a sheet, and includes one conductive member 92 and a thin insulating layer 94 supporting the conductive member 92. The conductive member 92 extends in parallel to the axis of the shape-memory member 20. The conductive member 92 is, for example, embedded in the insulating layer 94. The outside wire 90 is arranged to surround the outside of the inducing member 30. In FIG. 3, a pair of facing edges of the outside wire 90 are separated from each other, but may be in contact with each other. This layout enables the outside wire 90 to physically satisfactory protect the inducing member 30.

The inducing member 30 has a first end 32 located on a side of the first end 22 of the shape-memory member 20 and a second end 34 located on a side of the second end 24 of the shape-memory member 20.

The first end 32 of the inducing member 30 is electrically connected to the conductive member 92 of the outside wire 90 through a connector 96. The connector 96 may be formed by, for example, a wire, but is not limited thereto. As long as the connector 96 is a structure enabling electrical connection, it may be formed by calking, welding, brazing, soldering, conductive adhesion, or the like.

The second end 34 of the inducing member 30 is electrically connected to a controller 50 through a wire 62.

The conductive member 92 is electrically connected on a side of the second end 24 of the shape-memory member 20 to the controller 50 through a wire 64.

The controller 50 includes a power source 52 and a switch 54. The power source 52 and the switch 54 are connected in series. The switch 54 is connected to wire 64, and the power source 52 is connected to wire 62.

The variable-stiffness actuator 10B of the present embodiment is installed and operated in the same manner as that of the first embodiment.

### [Fourth Embodiment]

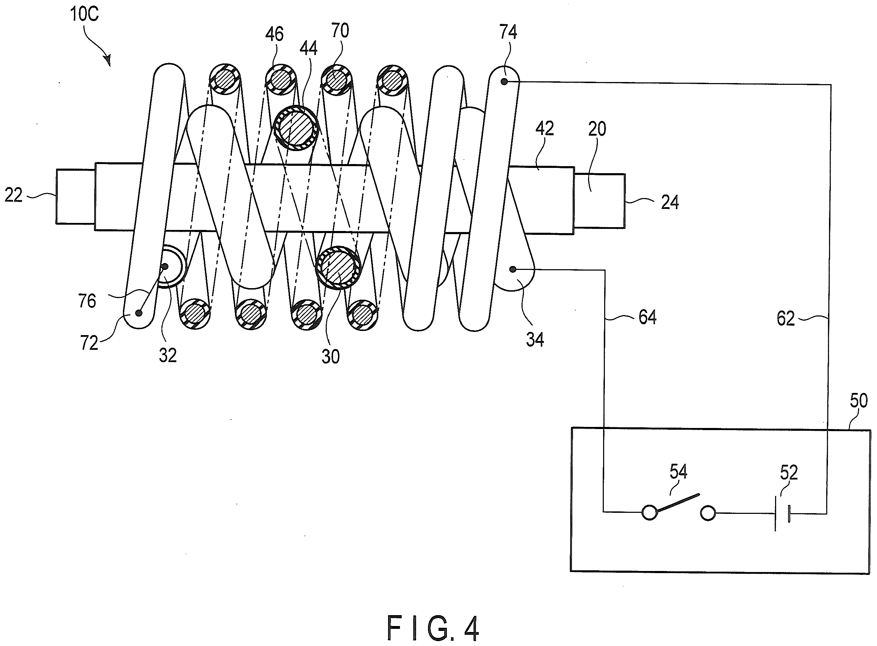

FIG. 4 shows a variable-stiffness actuator according to a fourth embodiment. In FIG. 4, the same members as those shown in FIG. 1 are assigned the same reference numerals as those shown in FIG. 1, and detailed descriptions thereof are omitted. The following descriptions will be provided while placing importance on the parts different from those in FIG. 1. Namely, the parts not described below are the same as those in the first embodiment.

The variable-stiffness actuator 10C of the present embodiment differs from the variable-stiffness actuator 10 of the first embodiment in that an insulating film 46 is provided around a conductive member 70.

Namely, as shown in FIG. 4, the variable-stiffness actuator 10C of the present embodiment includes a thin shape-memory member 20, an inducing member 30 arranged around the outside of the shape-memory member 20, and a conductive member 70 arranged around the outside of the inducing member 30. The inducing member 30 is electrically connected to the conductive member 70 through a connector 76. The inducing member 30 spirally extends around the shape-memory member 20 along the longitudinal axis of the shape-memory member 20. The conductive member 70 spirally extends around the inducing member 30 along the longitudinal axis of the shape-memory member 20.

An insulating film 46 is provided around the conductive member 70. The insulating film 46 serves to prevent a short circuit between the inducing member 30 and the conductive member 70 and a short circuit between adjacent portions of the conductive member 70.

The conductive member 70 and insulating film 46 constitute an outside wire shaped like a wire. This outside wire is arranged around the outside of the inducing member 30. The conductive member 70 spirally extends around the inducing member 30 along the longitudinal axis of the shape-memory member 20 with an appropriate clearance from the inducing member 30. This layout enables the outside wire to physically satisfactory protect the inducing member 30.

### [Fifth Embodiment]

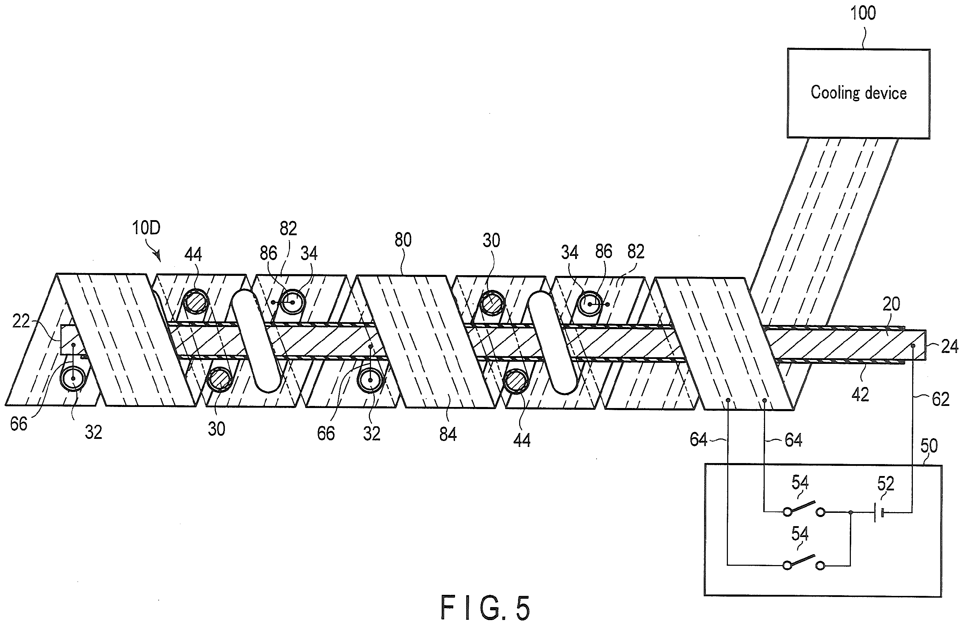

FIG. 5 shows a variable-stiffness actuator according to a fifth embodiment. In FIG. 5, the same members as those shown in FIG. 2 are assigned the same reference numerals as those shown in FIG. 2, and detailed descriptions thereof are omitted. The following descriptions will be provided while placing importance on the parts different from those in FIG. 2. Namely, the parts not described below are the same as those in the second embodiment.

Differing from the variable-stiffness actuator 10A of the second embodiment, the variable-stiffness actuator 10D of the present embodiment has a configuration in which an outside wire 80 is extended on a side of the second end 24 of the shape-memory member 20, and a cooling device 100 is connected to the extended outside wire 80, as shown in FIG. 5.

The cooling device 100 has a function of cooling a conductive member 82 of the outside wire 80. The cooling device 100 may be formed by, for example, a Peltier device, a heat radiation fin, a fan, or a water pipe.

The conductive member 82 of the outside wire 80 is not intended to heat the shape-memory member 20; therefore, it may be formed by a conductive member with small electrical resistance, in contrast to the inducing member 30. In general, a conductive member with small electrical resistance has high heat conductivity. Thus, the conductive member 82 is cooled well in response to the heat radiation action or cooling action of the cooling device 100. The conductive member 82 cooled by the cooling device 100 then cools the inducing member 30 and the shape-memory member 20. Accordingly, with the heat conductivity of the conductive member 82, the outside wire 80 contributes to cooling of the inducing member 30 and the shape-memory member 20. This contributes to reduction in time required for the shape-memory member 20 to return to the flexible state.

## Claims

1. A variable-stiffness actuator that is to be installed in a flexible member and configured to provide different stiffnesses to the flexible member, the variable stiffness actuator comprising:
a shape-memory member configured to transit in phase between a first phase and a second phase, the shape-memory member taking a flexible state in which the shape-memory member is easily deformable by an external force when the shape-memory member is in the first phase, so as to provide lower stiffness to the flexible member, and taking a rigid state in which the shape-memory member tends to take a memorized shape memorized beforehand against an external force when the shape-memory member is in the second phase, so as to provide higher stiffness to the flexible member;
a conductive inducing member configured to induce phase transition of the shape-memory member between the first phase and the second phase; and
a conductive member electrically connected to the inducing member, the conductive member being arranged around an outside of the inducing member and physically protecting the inducing member.

2. The variable-stiffness actuator according to claim 1, comprising an outside wire including the conductive member and an insulating layer provided around the conductive member.

3. The variable-stiffness actuator according to claim 2, wherein the outside wire is shaped like a belt.

4. The variable-stiffness actuator according to claim 3, wherein the outside wire is arranged so as to surround the outside of the inducing member.

5. The variable-stiffness actuator according to claim 3, wherein the outside wire spirally extends.

6. The variable-stiffness actuator according to claim 2, wherein, with heat conductivity of the conductive member, the outside wire contributes to cooling of the shape-memory member and the inducing member.

7. The variable-stiffness actuator according to claim 2, wherein the outside wire is shaped like a wire.

8. The variable-stiffness actuator according to claim 1, comprising a plurality of inducing members including the inducing member.

9. The variable-stiffness actuator according to claim 2, wherein the outside wire includes a plurality of conductive members including the conductive member.
